# DEMANDE DE BREVET EUROPEEN

(11) **EP 1 535 904 A2**
(43) Date de publication de la demande: **01.06.2005**
(21) Numéro de dépôt: 04106111.0
(22) Date de dépôt: 26.11.2004
(51) Int. Cl.: C07D 207/12, C07D 207/14, C07D 403/04, A61K 7/13, C09B 51/00, C09B 69/00

(54) **Procédé de préparation de dérives de 1- (4-nitrophényl)-3-pyrrolidinols optiquement actifs, et paraphénylenediamines à groupement pyrrolidinyle chirales**

(30) Priorité: 28.11.2003 FR 0350940
(71) Demandeur: L'OREAL, 75008 Paris (FR)
(72) Inventeur: Bordier, Thierry, 93290, Tremblay en France (FR); Xu, Jinzhu, 75014, Paris (FR)
(74) Mandataire: Poulin, Gérard

(57) **Abrégé**

Procédé de préparation d'un dérivé de 1-(4-nitrophényl)-3-pyrrolidinol optiquement actif répondant à la formule (I) suivante :

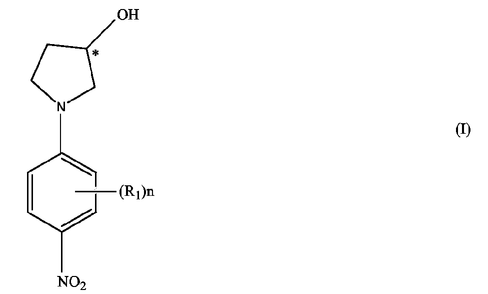

Dérivés de paraphénylènediamine substitués par un groupement pyrrolidinyle chiral, de formule (II) et leurs sels d'addition :

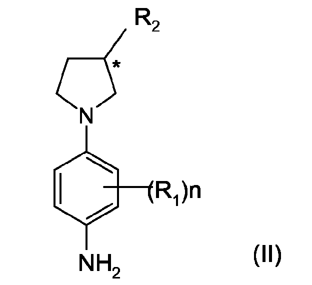

Composition tinctoriale contenant un dérivé de paraphénylènediamine de formule (II).

Procédé de teinture d'oxydation des fibres kératiniques mettant en oeuvre ladite composition.

Dispositifs à plusieurs compartiments dans lequel un premier compartiment contient ladite composition tinctoriale et un deuxième compartiment contient un agent oxydant.

## Description

L'invention concerne un procédé de préparation de dérivés de 1-(4-nitrophényl)-3-pyrrolidinols optiquement actifs.

L'invention concerne également de nouvelles paraphénylènediamines à groupement pyrrolidinyle chirales.

Les 1-(4-nitrophényl)-3-pyrrolidinols optiquement actifs répondent, par exemple, à la formule (I) suivante : 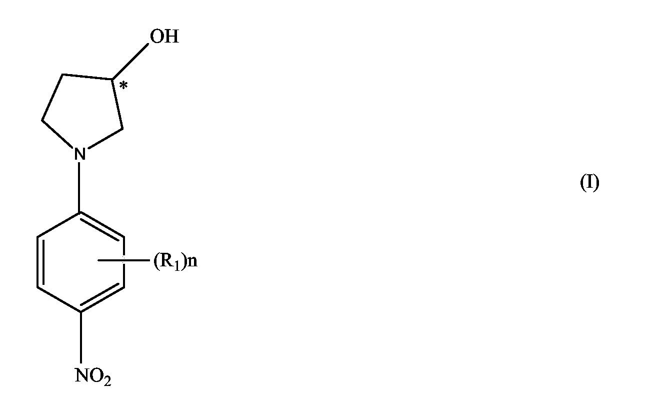 dans laquelle :
- le carbone portant le substituant OH est chiral (R ou S) ;
- n est compris entre 0 et 4, étant entendu que lorsque n est supérieur ou égal à 2, alors les radicaux R₁ peuvent être identiques ou différents ;
- R₁ représente un atome halogène ; une chaîne hydrocarbonée aliphatique, linéaire ou ramifiée, en C₁-C₈, ou alicyclique en C₃-C₈, saturée ou insaturée, un radical aryle, un radical arylalkyle dont la partie alkyle est en C₁-C₈, un ou plusieurs atomes de carbone de la chaîne hydrocarbonée et de la chaîne alkyle du radical arylalkyle pouvant être remplacé(s) par un atome d'oxygène, d'azote, de silicium, de soufre, ou par un groupement SO₂, le radical R₁ ne comportant pas de liaison peroxyde, ni de radicaux diazo, nitro ou nitroso.

Ces composés sont des précurseurs importants pour la synthèse des paraphénylènediamines à groupement pyrrolidinyle chiral substitués par un radical R₂ qui répondent à la formule (II) suivante : 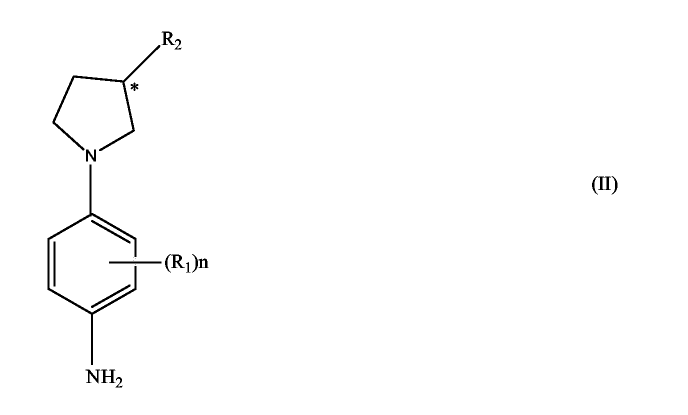 dans laquelle :
- le carbone portant le substituant R₂ est chiral (R ou S) ;
- n et R₁ ont la signification déjà donnée ci-dessus ; et
- R₂ représente un radical azoté cationique ou non.

Les composés de formule (II) sont utilisés comme base d'oxydation pour la teinture et dans les kits de teinture et ont l'avantage de présenter un bon profil toxicologique.

Certains composés de formule (I), dont le carbone portant le substituant OH sur le cycle pyrrolidine possède la configuration R, sont connus et leur synthèse a déjà été décrite, par contre, les composés de formule (I) ayant la configuration S ne sont pas connus.

Ainsi, le document WO-A-01 68043 décrit-il la préparation du composé (IV) par réaction entre 4-fluoro-nitrobenzène et le (R)-3-pyrrolidinol, selon le schéma 1 suivant :

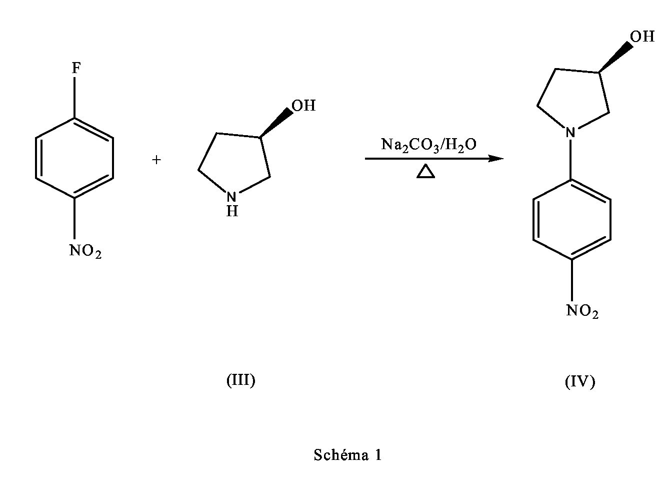

Les 3-pyrrolidinols chiraux (par exemple de formule (III), de configuration R ou S), qui sont les matières premières utilisées dans ce type de synthèse, sont des composés chers, difficiles à se procurer pour ce qui concerne les composés de configuration R, voire non accessibles industriellement pour ce qui concerne les composés de configuration S.

Plusieurs procédés de synthèse des (R) ou (S)-3-pyrrolidinols sont connus. Ces procédés sont en particulier les suivants :
1. Réduction par LiAlH₄ d'une (S)-3-hydroxy-pyrrolidone-2,5-dione N-substituée, obtenue par condensation de l'acide L-malique sur une amine primaire, ce qui conduit à la préparation du (S)-3-pyrrolidinol. Ce procédé est décrit dans EP-A-0 398 720 ; Synthetic Communication, 1985, 15(7), 587-98 ; et dans Journal of Medecinal Chemistry, 1994, 37, 2138-44.
2. Utilisation du 1,2,4-butanetriol chiral comme matière première en passant par un dérivé d'halogénure de cyclosulfinylalkyle. Ce procédé est décrit dans WO-A-00 15610.
3. Dédoublement chimique ou enzymatique des 3-pyrrolidinols racémiques. Ces procédés sont décrits dans JP-A-07 188124 et WO-A-09 503421.
4. Décarboxylation de la trans-4-hydroxy L-proline pour la synthèse de l'isomère R, selon le schéma 2 illustré ci-dessous. Dans plusieurs documents, ce procédé est décrit avec des protocoles différents pour isoler le produit final.

Le document Synthetic Communication, 1993, 23, 2691-9, décrit l'isolement de la forme neutre par distillation sous atmosphère réduite.

Le document WO-A-97 43256 décrit l'isolement de la forme chlorhydrate par précipitation avec l'acide chlorhydrique.

Le document Journal of Chemical Society Perkin Translation I, 1993, 1421, rapporte l'isolement du produit de décarboxylation par une précipitation avec l'acide maléique.

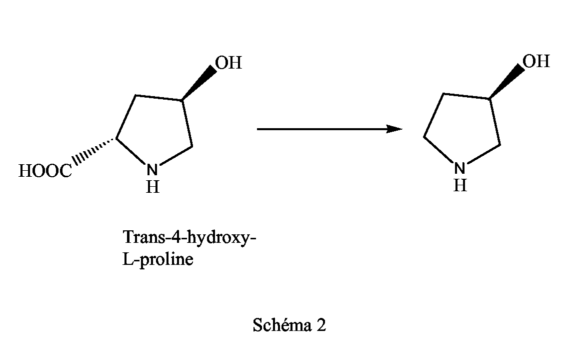

Tous les procédés mentionnés ci-dessus, à l'exception du quatrième mettent en oeuvre des procédés de synthèse longs et délicats, avec pour certains des matières premières ou réactifs chers.

La décarboxylation de la trans-4-hydroxy L-proline en (R)-3-pyrrolidinol (procédé 4) est le procédé le plus direct. Mais l'isolement du produit exige soit une distillation délicate soit une précipitation avec un acide. L'application industrielle peut se révéler délicate. De plus, ce procédé est limité à la préparation de l'isomère de configuration R.

Il ressort de ce qui précède qu'il existe un besoin pour un procédé de préparation de dérivés de 1-(4-nitrophényl)-3-pyrrolidinols optiquement actifs, chiraux, répondant notamment à la formule (I) ci-dessus qui soit facile à mettre en oeuvre, fiable, sûr, reproductible, et qui ne comporte qu'un nombre limité d'étapes simples.

Il existe encore un besoin pour un procédé de préparation de ces composés, qui utilise des matières premières ou produits de départ facilement disponibles et d'un faible coût, et qui permette d'obtenir les dérivés finaux de 1-(4-nitrophényl)-3-pyrrolidinols avec un rendement élevé.

Il existe en particulier un besoin pour un tel procédé qui évite d'utiliser en tant que matières premières des 3-pyrrolidinols qui sont des composés chers, peu ou pas disponibles et extrêmement longs et difficiles à synthétiser.

Le but de la présente invention est de fournir un procédé de préparation de dérivés de 1-(4-nitrophényl)-3-pyrrolidinols optiquement actifs qui réponde entre autres aux besoins énumérés plus haut.

Le but de la présente invention est encore de fournir un procédé de préparation de 1-(4-nitrophényl)-3-pyrrolidinols optiquement actifs qui ne présentent pas les inconvénients, défauts, limitations et désavantages des procédés de l'art antérieur et qui résolve les problèmes des procédés de l'art antérieur.

Ce but et d'autres encore sont atteints conformément à l'invention, dans une première variante, par un procédé de synthèse de préparation de dérivés de 1-(4-nitrophényl)-3-pyrrolidinols optiquement actifs répondant à la formule (I) suivante : 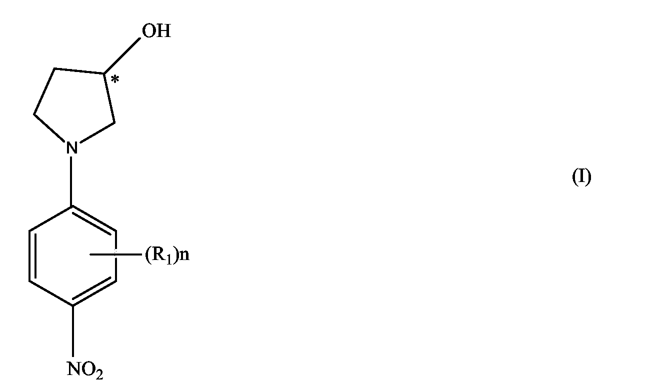 dans laquelle :
- le carbone portant le substituant OH sur le cycle pyrrolidine est chiral, et possède la configuration (R) ou (S) ;
- n est un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₈, de préférence en C₁-C₆, ou alicyclique en C₃-C₈, saturée ou insaturée, un radical aryle, un radical arylalkyle dont la partie alkyle est en C₁-C₈, de préférence en C₁-C₆, un ou plusieurs atomes de carbone de la chaîne hydrocarbonée et de la chaîne alkyle du radical arylalkyle pouvant être remplacé(s) par un atome d'oxygène, d'azote, de silicium, de soufre, ou par un groupement SO₂; le radical R₁ ne comportant pas de liaison peroxyde, ni de radicaux diazo, nitro ou nitroso ;
dans lequel on réalise les étapes successives suivantes :
a) on effectue la décarboxylation de la trans-4-hydroxy-L-proline : 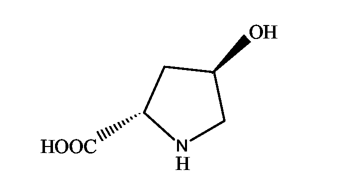 pour obtenir le (R)-3-pyrrolidinol : 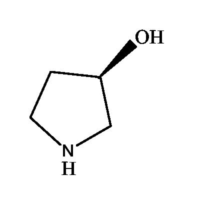
b) sans isolement, on fait réagir le (R)-3-pyrrolidinol formé, obtenu, avec un 4-halonitrobenzène de formule (V) : 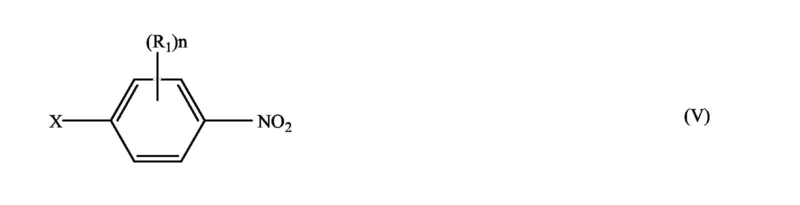 dans laquelle X représente un atome de F, Cl, Br ou I, et R₁ a la signification déjà donnée plus haut pour la formule (I), moyennant quoi on obtient le composé de formule (I) dans laquelle le carbone portant le substituant -OH sur le cycle pyrrolidine, possède la configuration (R) (composé I_{R}) : 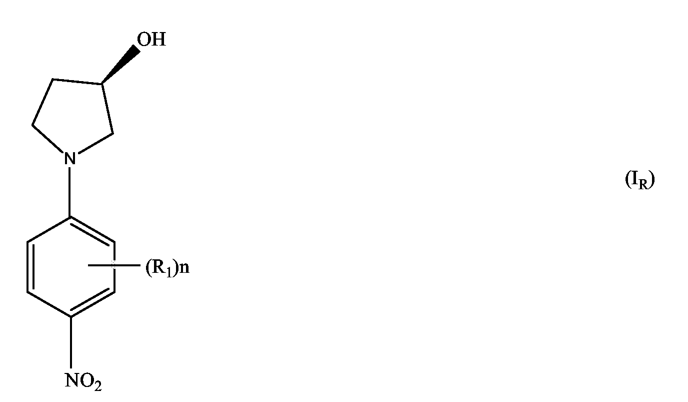
c) on réalise éventuellement une inversion de configuration du composé de formule (I_{R}), moyennant quoi, on obtient le composé de formule (I_{S}) :

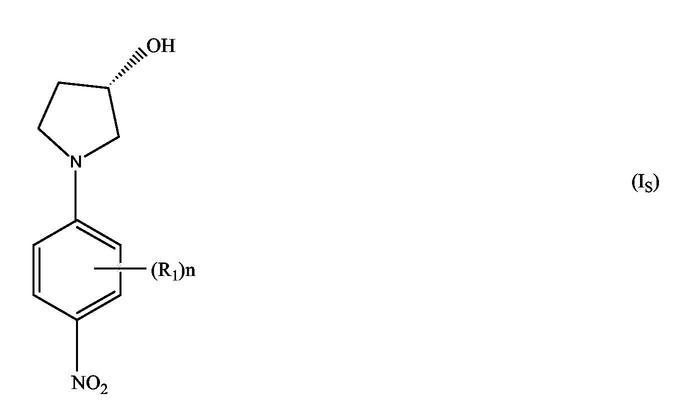

Selon une deuxième variante du procédé de l'invention, les buts susmentionnés et d'autres encore sont également atteints par un procédé de préparation d'un dérivé de 1-(4-nitrophényl)-3-pyrrolidinol optiquement actif de formule (I) : 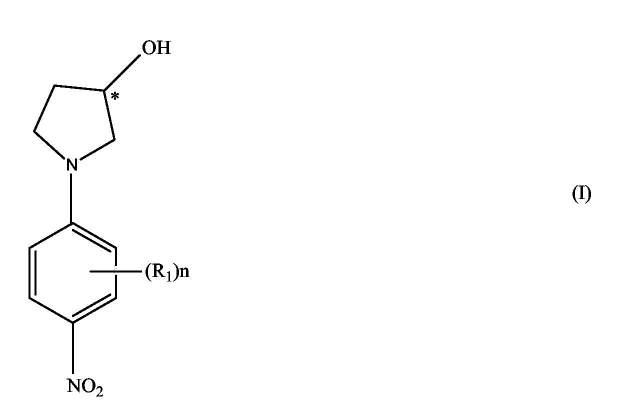 dans laquelle :
- le carbone portant le substituant -OH sur le cycle pyrrolidine est chiral, et possède la configuration (R) ou (S) ;
- n est un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₈, de préférence en C₁-C₆, ou alicyclique en C₃-C₈, saturée ou insaturée, un radical aryle, un radical arylalkyle dont la partie alkyle est en C₁-C₈, de préférence en C₁-C₆, un ou plusieurs atomes de carbone de la chaîne hydrocarbonée et de la chaîne alkyle du radical arylalkyle pouvant être remplacé(s) par un atome d'oxygène, d'azote, de silicium, de soufre, ou par un groupement SO₂; le radical R₁ ne comportant pas de liaisons peroxyde, ni de radicaux diazo, nitro ou nitroso ;
dans lequel on réalise les étapes successives suivantes :
a') on condense la trans-4-hydroxy-L-proline : 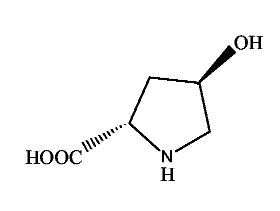 avec un 4-halonitrobenzène de formule (V) : 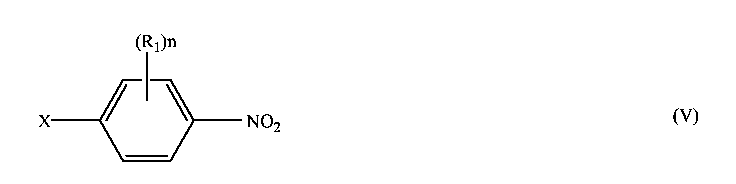 dans laquelle X représente un atome de F, Cl, Br ou I, et R₁ a la signification déjà donnée plus haut pour la formule (I), pour obtenir un composé de formule (VI) : 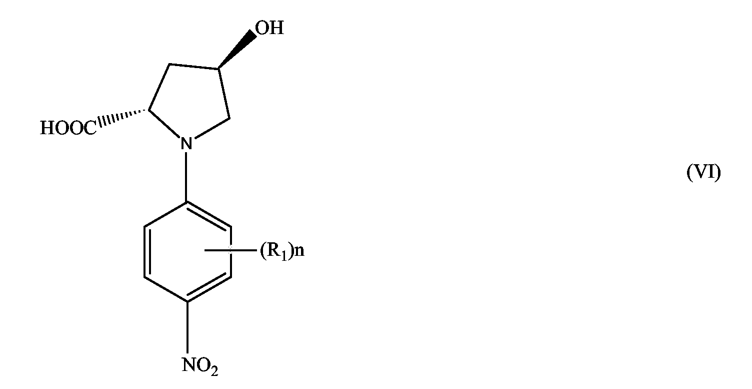
b') on effectue la décarboxylation du composé de formule (VI) pour obtenir le composé de formule (I) dans laquelle le carbone portant le substituant OH sur le cycle pyrrolidine possède la configuration (R) (composé I_{R}) : 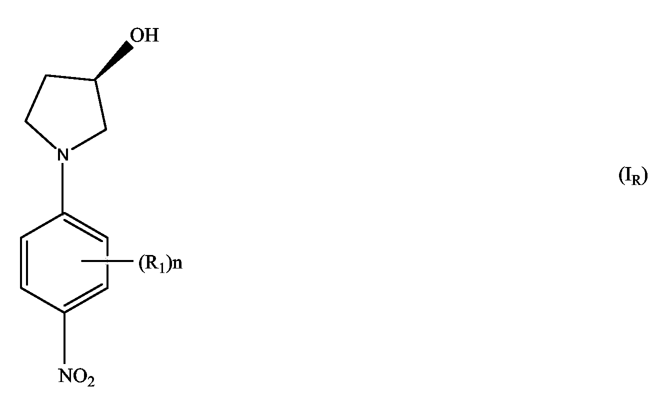
c') on réalise éventuellement une inversion de configuration du composé de formule (I_{R}), moyennant quoi on obtient un composé de formule (I_{S}) : 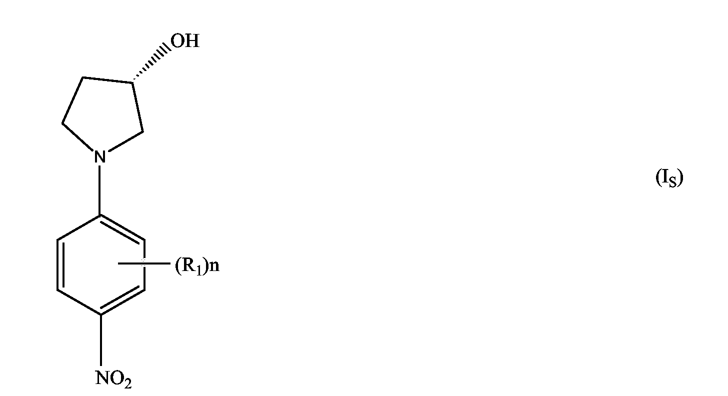
   Le procédé selon l'invention, aussi bien dans sa première que dans sa deuxième variante, fait appel à des produits de départ qui sont facilement disponibles industriellement, et d'un faible coût, puisqu'il s'agit essentiellement, d'une part, de la trans-4-hydroxy-L-proline et, d'autre part, des 4-halonitrobenzènes, qui sont des produits courants et peu onéreux.
   Le procédé selon l'invention ne fait pas appel, au contraire des procédés de l'art antérieur, à l'utilisation de 3-pyrolidinols chiraux en tant que matière première, de ce fait un des inconvénients majeurs des procédés de l'art antérieur est évité.
   Il est à noter que dans la première variante, du procédé selon l'invention, un (R)-3-pyrrolidinol est formé, mais il s'agit d'un produit intermédiaire et non d'un produit de départ et il n'est pas nécessaire d'isoler ce composé que l'on peut faire réagir directement avec le 4-halonitrobenzène.
   Dans la seconde variante du procédé selon l'invention, on n'a également pas besoin de préparer au préalable le (R)-3-pyrrolidinol.
   Le procédé selon l'invention, aussi bien dans sa première que dans sa seconde variante, est un procédé simple, fiable, qui présente un nombre limité d'étapes simples, faciles à mettre en oeuvre, et le rendement global du procédé est élevé.
   Le procédé selon l'invention donne accès aussi bien au composé dans lequel le carbone portant le substituant OH sur le cycle pyrrolidine se trouve dans la configuration (R), qu'au composé dans lequel cet atome de carbone se trouve dans la configuration (S).
   L'invention concerne, en outre, les nouveaux dérivés de paraphénylène diamines substitués par un groupement pyrrolidinyle chiral de formule (II) et leurs sels d'addition : 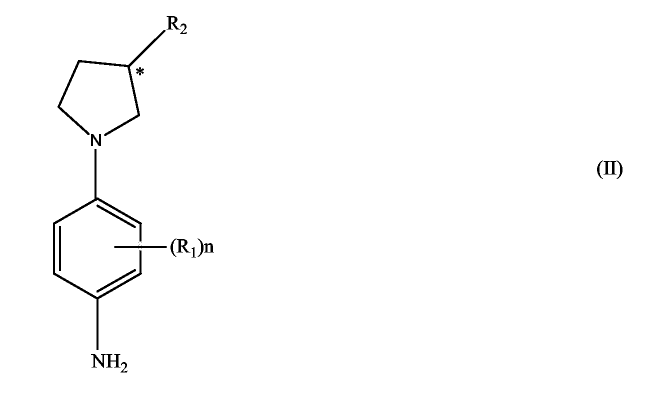 dans laquelle :
   - l'atome de carbone qui porte le substituant R₂ sur le cycle pyrrolidine est chiral et possède dans la configuration (R) (dérivé (II_{R})) ou (S) (dérivé (II_{S}) ) ;
   - R₁ et n ont la signification déjà donnée plus haut pour les dérivés de formule **(I)** ;
   - R₂ représente un radical azoté cationique ou non.

L'invention a aussi pour objet une composition tinctoriale contenant dans un milieu approprié pour la teinture de fibres kératiniques, au moins un dérivé de paraphénylènediamine de formule (II), dans une configuration (R) ou (S), à titre de base d'oxydation.

L'invention concerne également un procédé de teinture d'oxydation des fibres kératiniques dans lequel on applique sur les fibres une composition tinctoriale telle que définie ci-dessus, en présence d'un agent oxydant pendant un temps suffisant pour développer la coloration désirée.

Un autre objet de la présente invention est un dispositif à plusieurs compartiments dans lequel un premier compartiment contient une composition tinctoriale telle que définie ci-dessus et un deuxième compartiment contient un agent oxydant.

Un autre objet, encore, de l'invention est l'utilisation de cette composition pour la teinture de fibres kératiniques et un procédé de teinture de fibres kératiniques, en particulier les fibres kératiniques humaines telles que les cheveux, mettant en oeuvre la composition de la présente invention.

Les dérivés de paraphénylène diamines (PPD) substitués par un groupement pyrrolidinyle chiral de formule (II), issus des composés de formule (I) dont l'invention fait l'objet, sont utilisés comme base d'oxydation pour obtenir une coloration de fibres kératiniques chromatiques, puissante, peu sélective et tenace.

L'invention va maintenant être décrite de manière détaillée dans ce qui suit.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne cyclique, saturée ou insaturée, ne contenant pas de structure cyclique aromatique.

Lorsque la chaîne est interrompue par un atome Y d'oxygène, de soufre, d'azote, de silicium ou SO₂, on obtient, par exemple, un motif CH₂-Y-CH₂.

Dans la formule (I) , n peut être égal à 0 et le cycle benzénique ne porte alors pas de substituant. Dans le cas contraire, (n différent de 0) et à titre d'exemple, R₁ peut être un atome de chlore, un radical méthyle, éthyle, isopropyle, hydroxyméthyle méthoxyméthyle, hydroxyéthyle, 2-hydroxyéthyle, 3-hydroxypropyle, 1,2-dihydroxyéthyle, méthoxy, éthoxy, 2-hydroxyéthyloxy, phényle.

Dans la formule (I), lorsque n est différent de 0 et par exemple égal à 1, R₁ est de préférence un atome d'halogène ; une chaîne hydrocarbonée en C₁ à C₈, de préférence en C₁ à C₆, aliphatique, linéaire ou ramifiée, ou alicyclique en C₃ à C₈, saturée ou insaturée, un radical aryle, un radical arylalkyle dont la chaîne alkyle est en C₁-C₈, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

De préférence (n étant différent de 0 et par exemple égal à 1), R₁ est choisi parmi le chlore, le brome, un radical alkyle en C₁-C₆, avantageusement en C₁-C₄, hydroxyalkyle en C₁-C₄, , alcoxy en C₁-C₄, (C₁-C₄)-alcoxyalkyle en C₁-C₄, par exemple méthoxyméthyle, hydroxyalcoxy en C₁-C₄.

A titre d'exemple, R₁ est choisi parmi les radicaux méthyle, hydroxyméthyle, 2-hydroxyéthyle, 1,2-dihydroxyéthyle, méthoxy, isopropyloxy, 2-hydroxyéthoxy.

Les différentes étapes du procédé de l'invention selon sa première variante (voie A) ou selon sa seconde variante (voie B) sont illustrées de manière simplifiée sur le schéma 3 suivant :

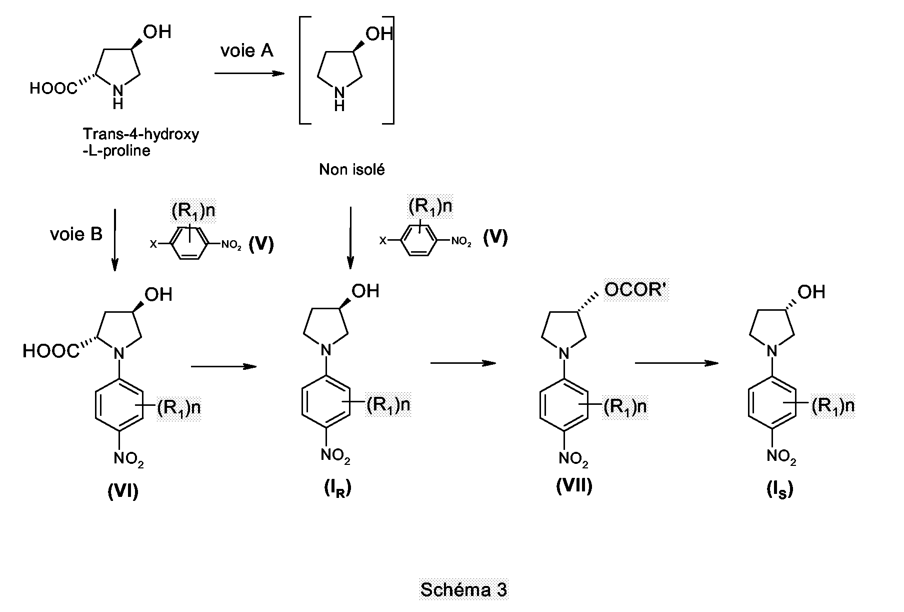

Dans la première variante du procédé selon l'invention, on effectue tout d'abord dans l'étape a), la décarboxylation de la trans-4-hydroxy-L-proline (voir schéma 3) pour donner le (R)-3-pyrrolidinol.

Cette décarboxylation est réalisée dans les conditions adéquates qui sont connues de l'homme du métier dans ce domaine de la technique.

La décarboxylation peut ainsi être réalisée de la manière suivante : on chauffe dans un solvant la trans-4-hydroxy-L-proline en présence d'un catalyseur tel que le cyclohexène-1-one. La température est généralement comprise entre 100 et 180°C. La réaction est en général mise en oeuvre en présence d'un solvant qui est habituellement choisi parmi le diméthylformamide, le diméthylsulfoxide, la N-méthylpyrolidinone ou certains alcools à point d'ébullition élevé tels que le cyclohexanol, le pentanol, le butanol.

A l'issue de cette étape a), le (R)-3-pyrrolidinol formé n'est pas séparé, c'est-à-dire qu'on le laisse dans le récipient ou réacteur où a eu lieu l'étape a) du procédé, et que l'on introduit dans ce récipient ou réacteur un 4-halonitrobenzène de formule (V), dans laquelle X représente un atome de F, Cl, Br ou I, et R₁ a la signification déjà donnée plus haut, pour qu'il réagisse avec le (R)-3-pyrrolidinol.

La réaction entre le (R)-3-pyrrolidinol et le 4-halonitrobenzène (V) est généralement réalisée de la manière suivante : on introduit le 4-halonitrobenzène et une base dans le mélange contenant le (R)-3-pyrrolidinol et on chauffe. La température de réaction est plus particulièrement comprise entre 75°C et 160°C. La base est notamment choisie parmi le carbonate de sodium, le bicarbonate de sodium, le carbonate de potassium, le bicarbonate de potassium, l'acétate de sodium et l'acétate de potassium.

Dans la deuxième variante du procédé selon l'invention (voie B sur le schéma 3), la première étape a') du procédé consiste à condenser la trans-4-hydroxy-L-proline, c'est-à-dire le même composé de départ que dans la première variante du procédé de l'invention sur un 4-halonitrobenzène de formule (V) pour former un composé de formule (VI).

Les conditions générale de cette réaction de condensation sont les suivantes : on chauffe dans un solvant du 4-halonitrobenzène et de la trans-4-hydroxy-L-proline en présence d'une base. La température est habituellement comprise entre 75 et 180°C. Le solvant est choisi plus particulièrement parmi le diméthylformamide, le diméthylsulfoxide, la N-méthylpyrolidinone, l'eau, les alcools tels que le propanol, l'isobutanol. La base est choisie de préférence parmi le carbonate de sodium, le bicarbonate de sodium, le carbonate de potassium, le bicarbonate de potassium, l'acétate de sodium et l'acétate de potassium.

Le composé de formule (VI) est ensuite décarboxylé (étape b')) pour donner le composé de formule (I) dans laquelle le carbone portant le substituant OH sur le cycle pyrrolidine possède la configuration (R) (composé I_{R}) .

La réaction de décarboxylation du composé (VI) est généralement réalisée de la manière suivante : on chauffe un mélange du composé de la formule (VI) avec un catalyseur tel que le cyclohexène-1-one dans un solvant. La température est généralement comprise entre 100 et 180°C. Le solvant est choisi plus particulièrement parmi le diméthylformamide, le diméthylsulfoxide, le N-méthylpyrolidinone, les alcools à point d'ébullition élevé tels que le cyclohexanol, le butanol, le pentanol, notamment.

A l'issue de l'étape b) de la première variante du procédé de l'invention, ou à l'issue de l'étape b') de la seconde variante du procédé de l'invention, on peut éventuellement, si l'on souhaite obtenir le composé de formule (I), dans laquelle le carbone portant le substituant OH sur le cycle pyrrolidine, qui possède la configuration (S) (composé I_{S}), réaliser une inversion de configuration du composé de formule (I_{R}) obtenu dans l'étape b) ou b') lors d'une étape optionnelle c), respectivement c').

Cette inversion de la configuration peut être réalisée par tout procédé d'inversion de configuration connu dans ce domaine de la technique.

On pourra, par exemple, effectuer une réaction de MITSUNOBU (voir les documents Bulletin of the Chemical Society of Japan, 1967, volume 40, 2380 , Australian Journal of Chemistry, 1988, volume 41, 1835 et Organic Préparations and Procédures International 1996, volume 28, 127) sur le composé de formule (I_{R}) pour obtenir un composé de formule (VII) : 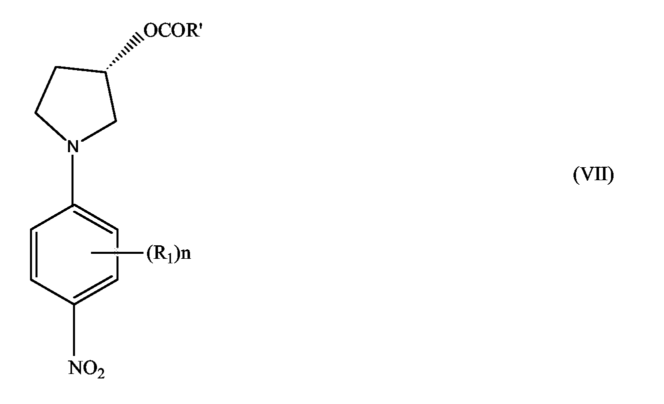 dans laquelle R₁ et n ont la signification déjà donnée plus haut et dans laquelle R' représente un atome d'hydrogène, un radical hydrocarboné en C₁-C₆ ou un radical aryle en C₆-C₈. Dans le composé de formule (VII), l'atome de carbone portant le groupe OCOR' se trouve dans la configuration (S).

Les conditions générales de la réaction de MITSUNOBU sont les suivantes : on ajoute de la triphénylphosphine et un acide carboxylique, R'COOH, à une solution contenant le composé de formule (I_{R}). Puis on ajoute du diéthyl azodicarbonate (EtOOCN=NCOOEt). Le produit formé de formule (VII) est ensuite isolé avec des techniques conventionnelles, par exemple la filtration ou l'extraction. L'acide carboxylique R'COOH est choisi notamment parmi l'acide acétique, l'acide benzoique, l'acide formique. Le solvant de réaction est en général un solvant éthérique, tel que le tétrahydrofurane, le diéthyléther, le méthyl tert-butyl éther.

Le composé de formule (VII) est ensuite hydrolysé dans les conditions générales suivantes : on traite le composé de formule (VII) avec une base choisie parmi les hydroxydes de métal alcalin comme la soude, la potasse ou l'hydroxyde de lithium pour donner un composé de formule (I_{S}) qui est isolé avec des techniques conventionnelles.

Les composés de formule (I), de configuration (R) ou (S), sont des précurseurs pour la synthèse des paraphénylènediamines de formule (II), portant un groupement pyrrolidine chiral et substituées par un radical R₂.

Cette synthèse est généralement réalisée de la manière suivante :
a) on active le composé de formule (I), (I_{R}) ou (I_{S}), pour former respectivement un composé de formule (III), (III_{R}) ou (III_{S}) : 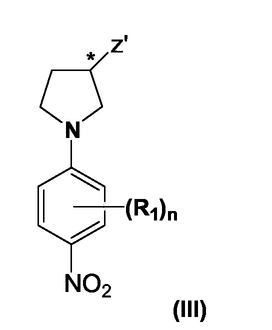 dans laquelle R₁ et n ont la signification déjà donnée plus haut et Z' représente un groupe partant ;
b) on fait réagir le composé de formule (III), (III_{R}) ou (III_{S}), avec une amine primaire, secondaire ou tertiaire, ou avec un composé portant un hétérocycle azoté aromatique
   pour former un composé de formule (IV), avec inversion de configuration (IV_{R}) ou (IV_{S}) : 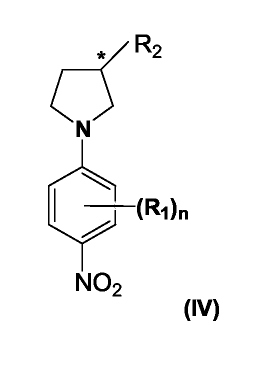
c) on transforme le groupement NO₂ en fonction amine par hydrogénation pour former le composé de formule (II), (II_{R}) ou (II_{S}) :

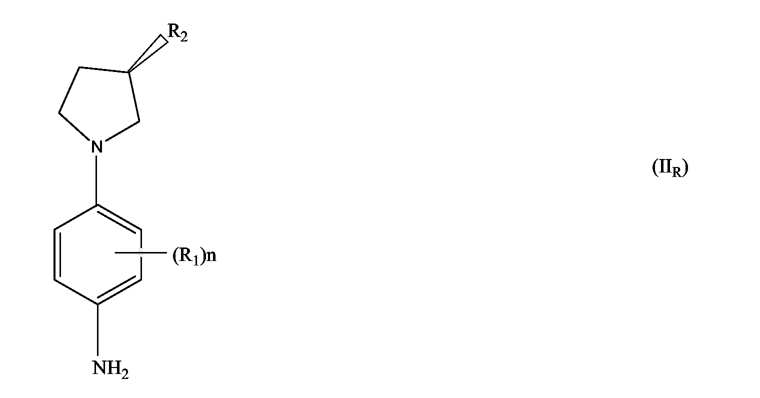

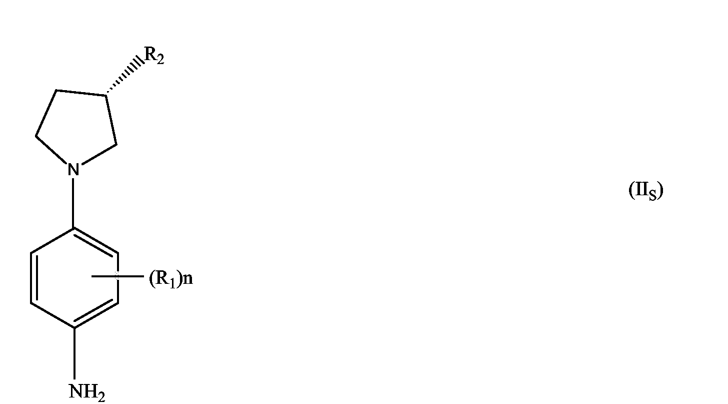

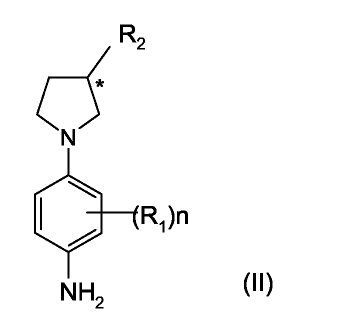

R₁ et n ont la signification (et les significations préférées) déjà donnée plus haut.

De même, comme indiqué auparavant, le radical R₂ représente un radical azoté cationique ou non cationique.

Si R₂ est un radical azoté cationique, il représente plus particulièrement un radical onium comme par exemple un radical ammonium, imidazolium, ou pyridinium.

Si R₂ est un radical azoté non cationique, il représente plus particulièrement un radical amine primaire (-NH₂), secondaire (-NHR) ou tertiaire (-NR₂) où R, identiques ou non, représentent un radical aliphatique, linéaire ou ramifié, saturé ou non, en C₁-C₂₂ , de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle.

Par ailleurs, les radicaux R peuvent former deux à deux, ensemble avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes. A titre d'exemples de tels cycles, on peut citer les cycles azétidine, pyrrolidine, pipéridine, pipérazine, morpholine, ledit hétérocycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl(C₁-C₆)carbonyle, un radical thio (-SH), un radical thioalkyle (-R-SH) en C₁-C₆, un radical alkyl(C₁-C₆)thio, un radical amino, un radical amino mono ou di-substitué par un radical alkyl (C₁-C₆) alkyl (C₁-C₆) carbonyle, amido ou alkyl(C₁-C₆)sulfonyle.

Conformément à un autre mode de réalisation, R₂ est un radical dérivé d'amino-guanidine (de formule -NH-NH-C (NH₂ ) =NH ) .

Si le radical R₂ de la formule (II) est un radical onium Z, il correspond dans un premier mode de réalisation à la formule (VIII) : 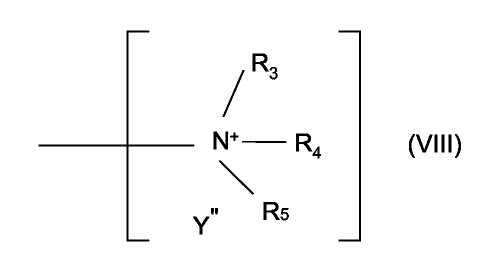 dans laquelle :
- R₃, R₄ et R₅, pris séparément, identiques ou non, représentent un atome d'hydrogène, un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) -sulfonyle ; ou
- R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes, à titre d'exemples de ce cycle, on peut citer les cycles azétidine, pyrrolidine, pipéridine, pipérazine ou morpholine, ledit hétérocycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl (C₁-C₆) carbonyle, un radical thio (-SH), un radical thioalkyle (-R-SH) en C₁-C₆, un radical alkyl(C₁-C₆)thio, un radical amino, un radical amino mono ou di-substitué par un radical alkyle (C₁-C₆) , alkyl (C₁-C₆) carbonyle, amido ou alkyl (C₁-C₆) sulfonyle ;
- Y" est un contre-ion.

Dans la formule (VIII), selon un mode plus particulier de réalisation, R₃, R₄ et R₅ séparément sont choisis de préférence parmi un radical alkyle en C₁-C₆, un radical monohydroxyalkyle en C₁-C₄, un radical polyhydroxyalkyle en C₂-C₄, un radical alcoxy (C₁-C₆) alkyle en C₁-C₄, un radical amidoalkyle en C₁-C₆, un radical trialkyl (C₁-C₆) silanealkyle en C₁-C₆ , ou R₃ avec R₄ forment ensemble un cycle azétidine, un cycle pyrrolidine, pipéridine, pipérazine, morpholine, R₅ étant choisi dans ce cas parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle mono ou di-substitué par un radical alkyl(C₁-C₆), alkyl (C₁-C₆) carbonyle, amido ou alkyl (C₁-C₆) sulfonyle ; un radical carbamylalkyle en C₁-C₆ ; un radical trialkyl (C₁-C₆) silanealkyle en C₁-C₆ ; un radical alkyl (C₁-C₆) carboxyalkyle en C₁-C₆ ; un radical alkyl (C₁-C₆) carbonyalkyle en C₁-C₆ ; un radical N-alkyl (C₁-C₆) carbamylalkyle en C₁-C₆.

Lorsque le radical R₂ correspond à la formule (VIII), il est de préférence un radical trialkylammonium dont les radicaux alkyles peuvent être substitués.

Selon un deuxième mode de réalisation, le radical R₂ représente un radical onium Z correspondant à la formule (IX) : 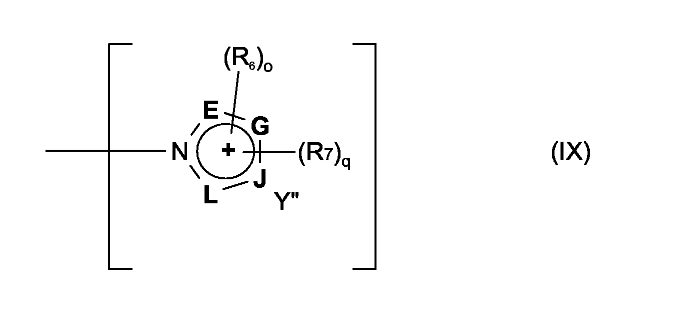 dans laquelle :
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
- q est un nombre entier compris entre 0 et 4 inclus ;
- o est un nombre entier compris entre 0 et 3 inclus ;
- q+o est un nombre entier compris entre 0 et 4 inclus ;
- R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
R₆, identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical benzyle ; étant entendu que les radicaux R₆ sont portés par un azote ;
- Y" est un contre-ion.

A titre d'exemple, les sommets E, G, J et L peuvent former un cycle pyrrolique, imidazolique, pyrazolique, oxazolique, thiazolique ou triazolique, de préférence imidazolique.

Selon un troisième mode de réalisation, R₂ représente le radical onium Z correspondant à la formule (X) : 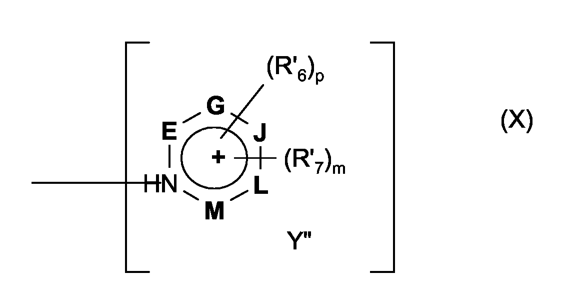 dans laquelle :
- les sommets E, G, J, L et M identiques ou différents, représentent un atome de carbone, d'oxygène, de soufre ou d'azote pour former un cycle choisi parmi les cycles pyridiniques, pyrimidiniques, pyraziniques, triaziniques et pyridaziniques ;
- p est un nombre entier compris entre 0 et 3 inclus ;
- m est un nombre entier compris entre 0 et 5 inclus ;
- p+m est un nombre entier compris entre 0 et 5 inclus ;
- R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ étant entendu que les radicaux R'₇ sont portés par un atome de carbone ;
- R'₆ , identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical benzyle ; étant entendu que les radicaux R'₆ sont portés par un azote ;
- Y" représente un contre-ion.

De préférence, les sommets E, G, J, L et M forment avec l'azote du cycle un cycle pyridinique ou pyrimidinique.

De préférence, R'₇ et R'₆ de même que R₇ et R₆ sont des radicaux alkyles pouvant être substitués.

Dans le cadre de l'invention, le contre-ion (Y") peut être choisi parmi un atome d'halogène tel que le brome, le chlore, le fluor ou l'iode, un hydroxyde, un citrate, un succinate, un tartrate, un lactate, un tosylate, un mésylate, un benzènesulfonate, un acétate, un hydrogènesulfate ou un alkylsulfate en C₁-C₆ tel que par exemple le méthylsulfate, ou l'éthylsulfate.

De préférence, le contre-ion (Y") est choisi parmi un atome d'halogène tel que le brome, le chlore ou l'iode, un tosylate, un mésylate, un benzènesulfonate, ou un alkylsulfate en C₁-C₆ tel que par exemple le méthylsulfate, ou l'éthylsulfate.

Des exemples de composés de formule (II) qui peuvent être préparés par le procédé de l'invention sont les suivants :

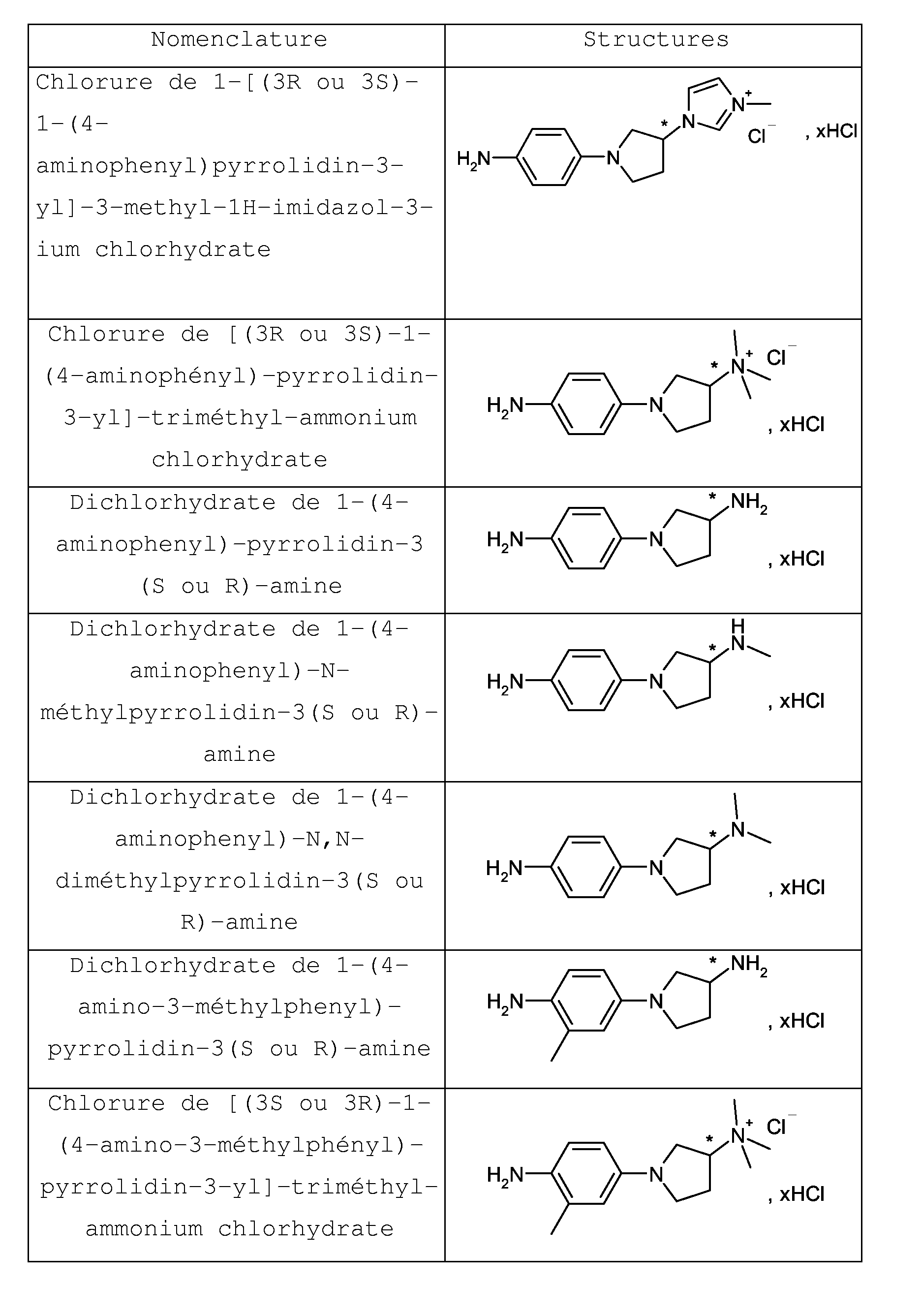

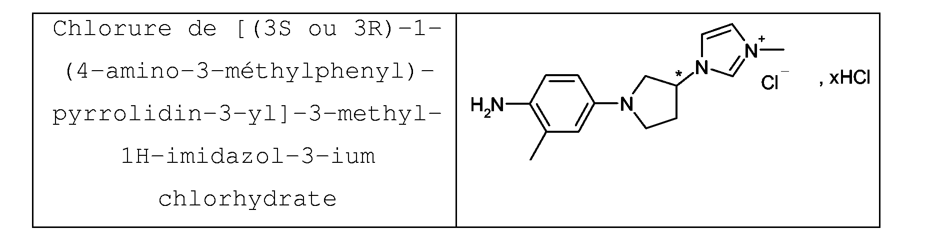

La composition tinctoriale de la présente invention comprend, dans un milieu approprié pour la teinture des fibres kératiniques, en particulier les fibres kératiniques humaines telles que les cheveux humains, à titre de base d'oxydation un dérivé de formule (II), dans la configuration (R) ou (S), tel que défini précédemment.

La ou les bases d'oxydation de l'invention sont en général présentes chacune en quantité comprise entre 0,001 à 10 % en poids environ du poids total de la composition tinctoriale, de préférence entre 0,005 et 6 % en poids.

La composition tinctoriale de l'invention peut contenir en outre un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(-β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment.

La quantité de chacune des bases d'oxydation additionnelles est généralement comprise entre 0,001 et 10 % en poids environ du poids total de la composition tinctoriale.

A titre d'exemple, ces bases d'oxydation additionnelles sont choisies parmi les paraphénylènediamines autres que celles décrites précédemment, les bis-phénylalkylènediamines, les para-aminophénols, les bis-paraaminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 4-N,N-bis(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxyéthyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxopropyl) paraphénylènediamine, la N-(4'-aminophényl) paraphénylènediamine, la N-phényl paraphénylènediamine, la 2-β-hydroxyéthyoxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β-hydroxyéthylamino 5-amino toluène, la 3-hydroxy 1-(4'-aminophényl)pyrrolidine et leurs sels d'addition avec un acide sont préférées.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB-A-1 026 978 et GB-A-1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR-A-2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo [1, 5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE-A-2359399 ; JP-A-88-169571 ; JP-A-05-63124 ; EP-A-0770375 ou la demande de brevet WO-A-96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE-A-3843892, DE-A-4133957 et les demandes de brevet WO-A-94/08969, WO-A-94/08970, FR-A-2 733 749 et DE-A-195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 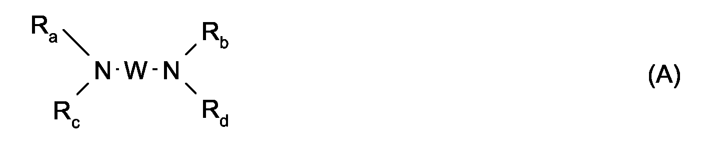 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, plus particulièrement humaines et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps suffisant pour développer la coloration désirée, dit temps de pose qui est par exemple de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme, contient la composition tinctoriale de la présente invention définie ci-dessus et un deuxième compartiment renferme, contient une composition, avec agent oxydant. Ce dispositif peut être équipé d'un moyen permettant de délivrer sur les cheveux le mélange souhaité, tel que les dispositifs décrits dans le brevet FR-A-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (II), dans la configuration (R) ou (S), avec un agent oxydant, et l'application du mélange obtenu sur les fibres kératiniques pendant un temps suffisant pour développer la coloration désirée.

L'invention va maintenant être décrite en référence à l'exemple suivant, donné à titre illustratif et non limitatif.

### Exemple 1

Le (3R)-1-(4-nitrophényl)-pyrrolidin-3-ol, composé de formule (I) ((I_{R})), dans laquelle R₁ est l'hydrogène est préparé par le procédé selon l'invention dans sa première variante, selon le schéma 4 suivant :

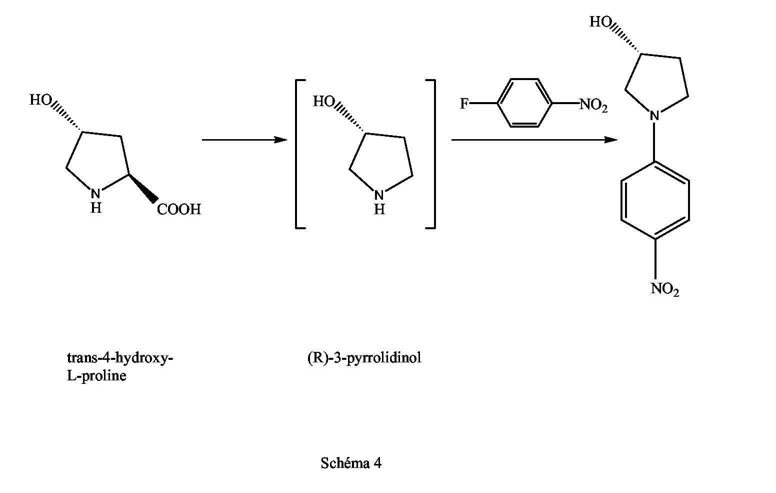

Dans un réacteur, sous atmosphère d'azote, on introduit 100 g (0,763 moles) de trans 4-hydroxy-L-proline dans 500 ml de N-méthyl-pyrrolidone et 5 ml de cyclohexène-1-one.

On chauffe à 155°C pendant 3 h, puis on refroidit à la température ambiante.
On introduit 126,5 g (0,915 moles) de carbonate de potassium, puis on laisse s'écouler dans le réacteur 96,8 g (0,686 moles) de 4-fluoro-1-nitrobenzène en solution dans 125 ml de N-méthyl-pyrrolidone. On chauffe à 105°C pendant 3 heures. On refroidit à 10°C, puis on verse dans un grand volume d'eau glacée. On filtre le précipité. On le lave à l'eau, puis on le sèche sous vide à 50°C. On obtient ainsi 127,5 g d'un solide jaune, soit un rendement final de 89 %. Le solide obtenu est caractérisé par les analyses suivantes.

### Analyses

Les spectres de masse et de RMN 1H et 13C sont conformes à la structure attendue.

Point de fusion (DSC) : 180,7°C.

| Analyse élémentaire (C12H16N202 : PM = 220,27) . | | | | |
|---|---|---|---|---|
| | C | H | N | O |
| % théorique | 57,69 | 5,81 | 13,45 | 23,05 |
| % trouvé | 57,55 | 5,82 | 13,46 | 22,84 |

Le chlorure de 1-[(3S)-1-(4-aminophényl)pyrrolidin-3-yl]-3-méthyl-1H-imidazol-3-ium chlorhydrate, composé de formule (II) ((II_{S})), dans laquelle R₁ est l'hydrogène et R₂ représente un radical azoté cationique est préparé par le procédé selon le schéma suivant :

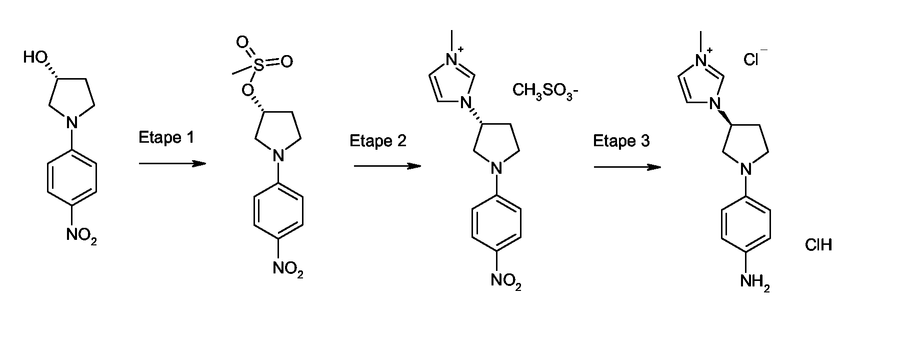

### Etape 1 :

### (3R)-1-(4-nitrophényl)pyrrolidin-3-yl méthanesulfonate

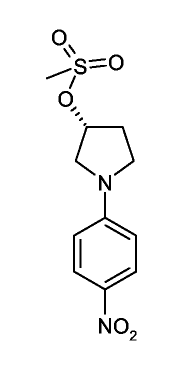

Dans un réacteur sous atmosphère d'azote, on introduit 75.8 g (0.364 moles) du composé de l'étape précédente dans 580 ml de pyridine. On refroidit à 0°C puis on ajoute 33.9 ml (0,437 moles) de chlorure de méthanesulfonyle en maintenant la température inférieure à 5°C.

On agite à 5°C pendant 3h45 puis on verse le mélange dans 5.25 L d'eau.

On filtre, on rince le solide à l'eau puis on sèche sous vide à 40°C.

On obtient 79 g d'un solide beige rosé soit un rendement final de 76%.

### Analyses

Les spectres de masse et de RMN 1H et 13C sont conformes à la structure attendue.

### Etape 2 :

### 3-méthyl-1-[(3S)-1-(4-nitrophényl)pyrrolidin-3-yl]-1H-imidazol-3-ium méthanesulfonate

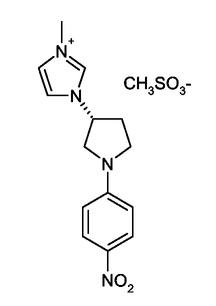

Dans un réacteur sous atmosphère d'azote, on introduit 200 g (0,67 moles) du composé précédent dans 2000 ml de méthylisobutylcétone. On porte le mélange à 80°C puis on ajoute goutte à goutte 211 ml de N-méthylimidazole. On chauffe ensuite au reflux à 115°C pendant 6h, on refroidit à 20°C puis on filtre.

On lave le solide à l'acétone puis on sèche sous vide.

On obtient 188.3 g d'un solide beige clair soit un rendement de 73%.

### Analyses

Les spectres de masse et de RMN 1H et 13C sont conformes à la structure attendue.

### Etape 3 :

### Chlorure de 1-[(3S)-1-(4-aminophényl)pyrrolidin-3-yl]-3-méthyl-1H-imidazol-3-ium chlorhydrate

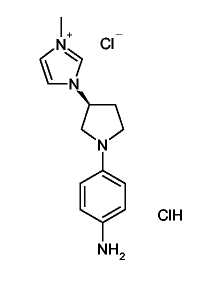

Dans un réacteur à hydrogéner en inox, on dissout partiellement 12 g (32.57 mmol) du composé de l'étape précédente dans 200 ml d'éthanol 96. On ajoute 1.2 g de Pd/C à 5 % (50% humide), on ferme le réacteur et le purge à l'azote 3 fois sous agitation (1800 tour/min). On introduit ensuite l'hydrogène sous une pression de 5-6 bars à température ambiante. La température monte jusqu'à 31°C puis, au bout d'1h15 redescend à 27°C.

Le réacteur est alors purgé à l'azote et le mélange réactionnel est filtré sous atmosphère d'azote. Le filtrat est récupéré immédiatement dans une solution contenant 13.5 ml d'acide chlorhydrique à 37% et 20 ml d'éthanol à 96.

Le filtrat est ensuite évaporé jusqu'à l'obtention d'un précipité.

Le solide est filtré, lavé à l'isopropanol puis à l'éther éthylique et séché sous vide en présence de potasse.

On obtient ainsi 5.1 g d'un solide blanc soit un rendement de 49%.

### Analyses

Les spectres de masse et de RMN 1H et 13C sont conformes à la structure attendue.

## Revendications

1. Procédé de préparation d'un dérivé de 1-(4-nitrophényl)-3-pyrrolidinol optiquement actif répondant à la formule (I) suivante : 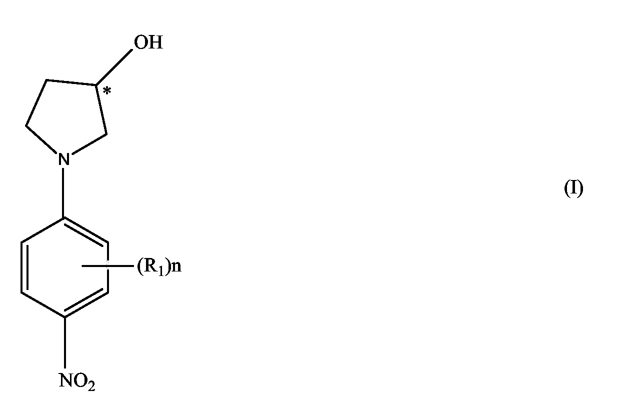 dans laquelle :
- le carbone portant le substituant -OH sur le cycle pyrrolidine est chiral, et possède la configuration (R) ou (S) ;
- n représente un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₈, de préférence en C₁-C₆, ou alicyclique en C₃-C₈, saturée ou insaturée, un radical aryle, un radical arylalkyle dont la partie alkyle est en C₁-C₈, de préférence en C₁-C₆, un ou plusieurs atomes de carbone de la chaîne hydrocarbonée et de la chaîne alkyle du radical arylalkyle pouvant être remplacé(s) par un atome d'oxygène, d'azote, de silicium, de soufre, ou par un groupement SO₂ ; le radical R₁ ne comportant pas de liaison peroxyde, ni de radicaux diazo, nitro ou nitroso,
dans lequel on réalise les étapes successives suivantes :
a) on effectue la décarboxylation de la trans-4-hydroxy-L-proline : 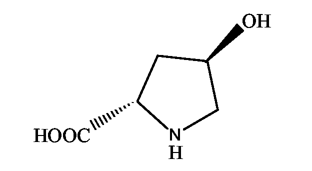 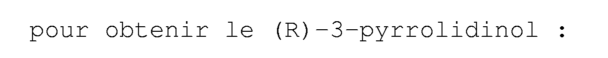 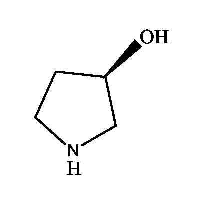
b) sans isolement, on fait réagir le (R)-3-pyrrolidinol obtenu avec un 4-halonitrobenzène de formule (V) : 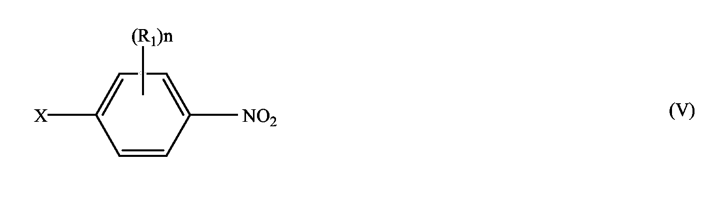 dans laquelle X représente un atome de F, Cl, Br ou I, et R₁ a la signification déjà donnée plus haut pour la formule (I), moyennant quoi on obtient le composé de formule (I) dans laquelle le carbone portant le substituant -OH sur le cycle pyrrolidine, possède la configuration (R) (composé I_{R}) : 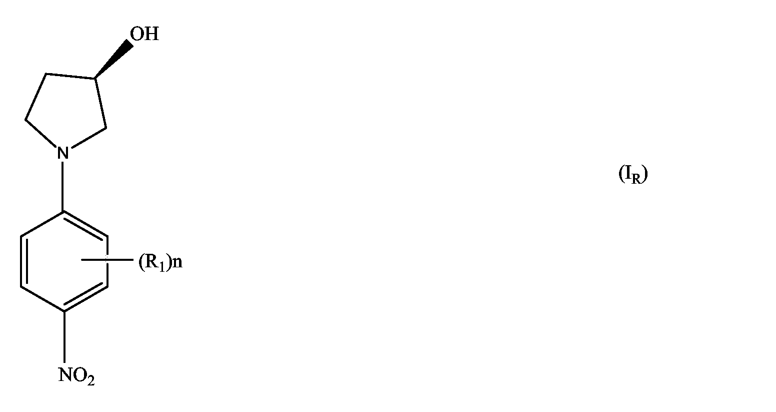
c) on réalise éventuellement une inversion de configuration du composé de formule (I_{R}), moyennant quoi, on obtient le composé de formule (I_{S}) :

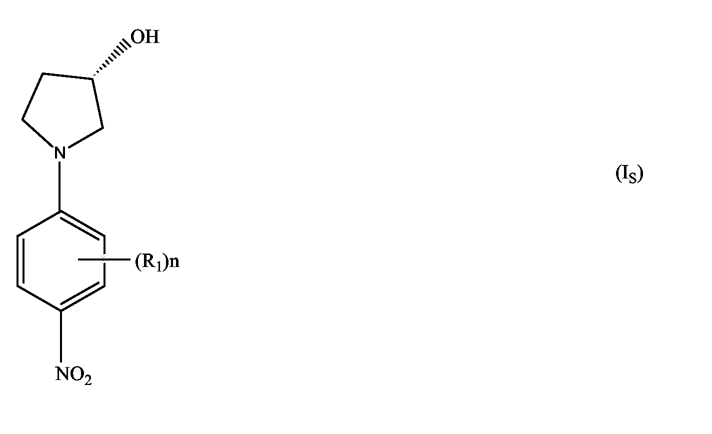

2. Procédé de préparation d'un dérivé de 1-(4-nitrophényl)-3-pyrrolidinol optiquement actif de formule (I) : 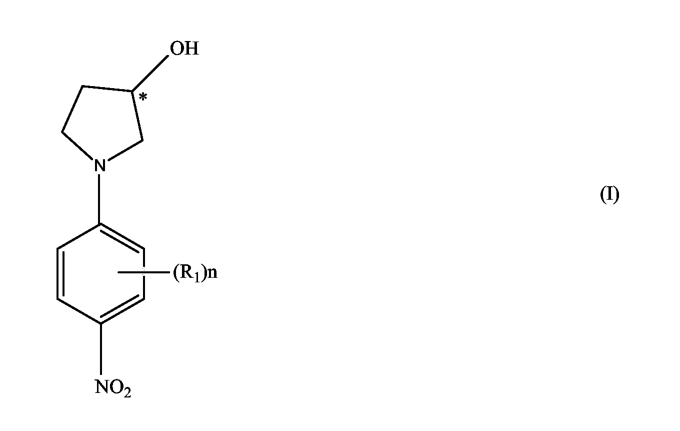 dans laquelle :
- le carbone portant le substituant -OH sur le cycle pyrrolidine est chiral, et possède la configuration (R) ou (S) ;
- n représente un nombre entier de 0 à 4, étant entendu que lorsque n est supérieur ou égal à 2, alors les radicaux R₁ peuvent être identiques ou différents ;
- R₁ représente un atome d'halogène ; une chaîne hydrocarbonée aliphatique, linéaire ou ramifiée, en C₁-C₈, de préférence en C₁-C₆, ou alicyclique en C₃-C₈, saturée ou insaturée, un radical aryle, un radical arylalkyle dont la partie alkyle est en C₁-C₈, de préférence en C₁-C₆, un ou plusieurs atomes de carbone de la chaîne hydrocarbonée et de la chaîne alkyle du radical arylalkyle pouvant être remplacé(s) par un atome d'oxygène, d'azote, de silicium, de soufre, ou par un groupement SO₂, le radical R₁ ne comportant pas de liaison peroxyde, ni de radicaux diazo, nitro ou nitroso,
dans lequel on réalise les étapes successives suivantes :
a') on condense la trans-4-hydroxy-L-proline : 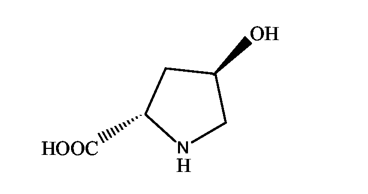
avec un 4-halonitrobenzène de formule (V) : 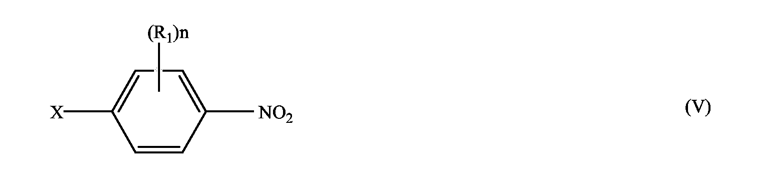 dans laquelle X représente un atome de F, Cl, Br ou I, et R₁ a la signification déjà donnée plus haut pour la formule (I), pour obtenir un composé de formule (VI) : 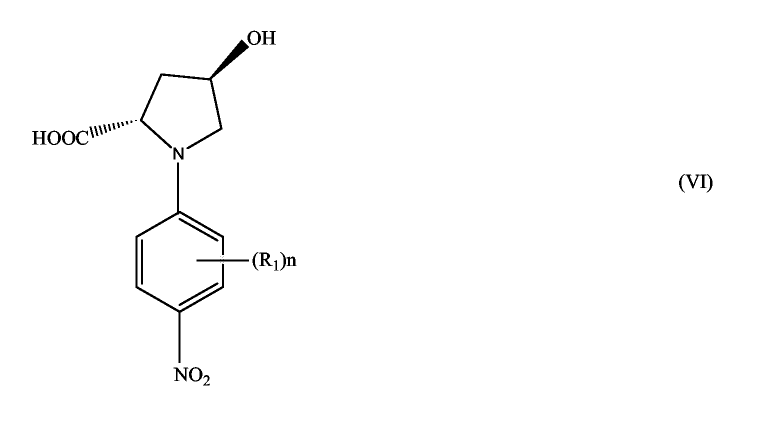
b') on effectue la décarboxylation du composé de formule (VI) pour obtenir le composé de formule (I) dans laquelle le carbone portant le substituant OH sur le cycle pyrrolidine possède la configuration (R) (composé I_{R}) : 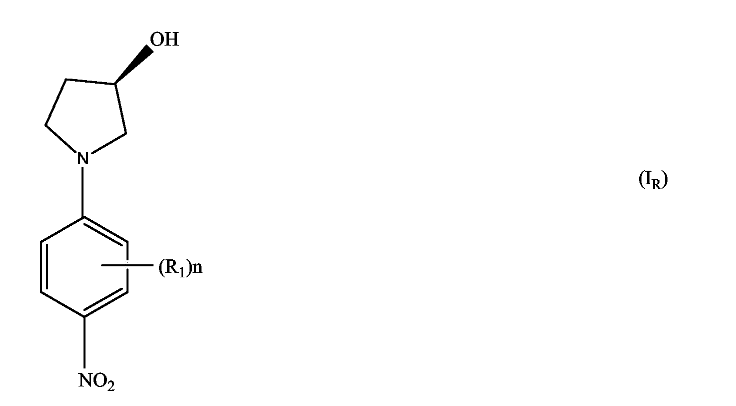
c') on réalise éventuellement une inversion de configuration du composé de formule (I_{R}), moyennant quoi on obtient un composé de formule (I_{S}) :

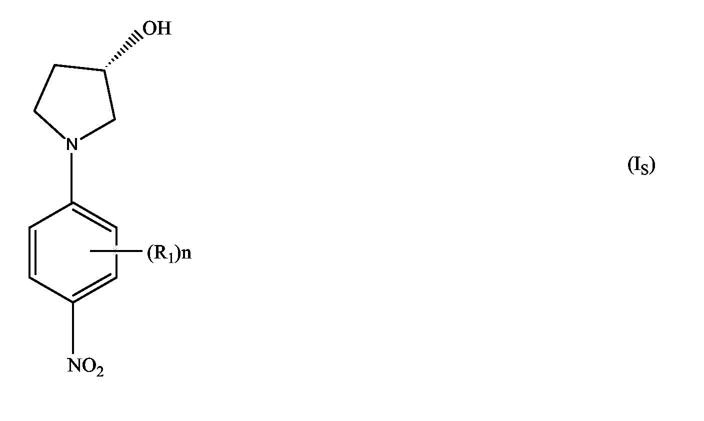

3. Procédé selon la revendication 1 ou 2, dans lequel n est égal à 0.

4. Procédé selon la revendication 1 ou 2, dans lequel n est différent de 0 et par exemple égal à 1, et R₁ est un atome d'halogène ; une chaîne hydrocarbonée en C₁-C₈, de préférence en C₁-C₆ aliphatique, linéaire ou ramifiée, ou alicyclique en C₃-C₈, saturée ou insaturée, un radical aryle, un radical arylalkyle dont la chaîne alkyle est en C₁-C₈, de préférence en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, ou par un groupement SO₂ ; le radical R₁ ne comportant pas de liaison peroxyde, ni de radicaux diazo, nitro ou nitroso.

5. Procédé selon l'une quelconque des revendications 1, 2 et 4, dans lequel R₁ est choisi parmi, le chlore, le brome, un radical alkyle en C₁-C₆ avantageusement en C₁-C₄, un radical (C₁-C₄) alcoxy alkyle en C₁-C₄, un radical hydroxyalkyle en C₁-C₄, alcoxy en C₁-C₄, hydroxyalcoxy en C₁-C₄.

6. Procédé selon la revendication 5, dans lequel R₁ est choisi parmi les radicaux méthyle, hydroxyméthyle, 2-hydroxyéthyle, 1,2-dihydroxyéthyle, méthoxy, isopropyloxy, 2-hydroxyéthoxy.

7. Procédé selon la revendication 1 ou 2, dans lequel l'inversion de configuration du composé de formule (I_{R}) dans l'étape c) ou c') est réalisée en effectuant une réaction de MITSUNOBU sur le composé de formule (I_{R}) pour obtenir un composé de formule (VII) : 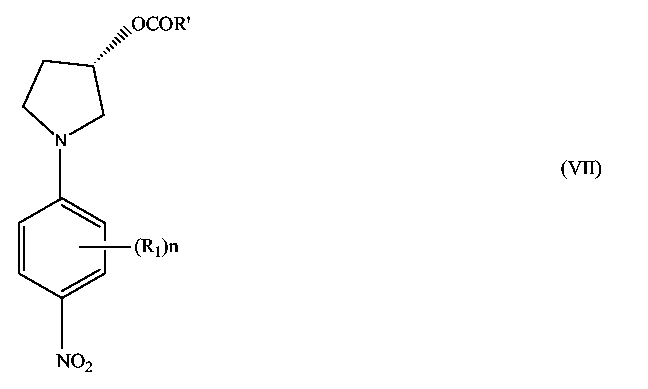 dans laquelle R₁ et n ont la signification déjà donnée dans la revendication 1 ou la revendication 2, et R' représente un atome d'hydrogène, un radical hydrocarboné en C₁-C₆ ou un radical aryle en C₆-C₈, puis en hydrolysant le composé de formule (VII) pour donner le composé de formule (I_{S}) .

8. Dérivés de paraphénylènediamine substitués par un groupement pyrrolidinyle chiral, de formule (II), susceptibles d'être préparés à partir d'un dérivé intermédiaire de formule (I) tel que décrit dans la revendication 1, et leurs sels d'addition : 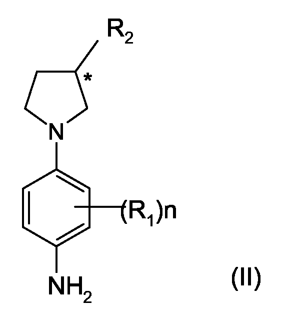 dans laquelle :
- l'atome de carbone qui porte le substituant R₂ sur le cycle pyrrolidine est chiral et possède la configuration (R) ou (S) ;
- n représente un nombre entier de 0 à 4, étant entendu que lorsque n est supérieur ou égal à 2, alors les radicaux R₁ peuvent être identiques ou différents ;
• R₁ représente un atome d'halogène ; une chaîne hydrocarbonée aliphatique, linéaire ou ramifiée, en C₁-C₈, de préférence en C₁-C₆, ou alicyclique en C₃-C₈, saturée ou insaturée, un radical aryle, un radical arylalkyle dont la partie alkyle est en C₁-C₈, de préférence en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, par un groupement SO₂, ; le radical R₁ ne comportant pas de liaison peroxyde, ni de radicaux diazo, nitro, ou nitroso ;
• R₂ représente un radical azoté cationique ou non.

9. Dérivés selon la revendication 8 dans lesquels n est égal à 0.

10. Dérivés selon la revendication 8 dans lesquels n est différent de 0 et par exemple égal à 1, et R₁ est un atome d'halogène ; une chaîne hydrocarbonée en C₁-C₈, de préférence en C₁-C₆, aliphatique, linéaire ou ramifiée,ou alicyclique en C₃-C₈, saturée ou insaturée, un radical aryle, un radical arylalkyle dont la chaîne alkyle est en C₁-C₈, de préférence en C₁-C₆, un ou plusieurs atomes de carbone de la chaîne hydrocarbonée et de la chaîne alkyle du radical arylalkyle pouvant être remplacés par un atome d'oxygène, d'azote, de silicium, de soufre, par un groupement SO₂ ; le radical R₁ ne comportant pas de liaison peroxyde, ni de radicaux diazo, nitro ou nitroso.

11. Dérivés selon la revendication 10 dans lesquels R₁ est choisi parmi le chlore, le brome, un radical alkyle en C₁-C₆, avantageusement en C₁-C₄, un radical hydroxyalkyle en C₁-C₄, un radical (C₁-C₄) alcoxy alkyle en C₁-C₄, un radical alcoxy en C₁-C₄, un radical hydroxyalcoxy en C₁-C₄.

12. Dérivés selon la revendication 11 dans lesquels R₁ est choisi parmi les radicaux méthyle, hydroxyméthyle, 2-hydroxyéthyle, 1,2-dihydroxyéthyle, méthoxy, isopropyloxy, 2-hydroxyéthoxy.

13. Dérivés selon l'une quelconque des revendications 8 à 12, dans lesquels R₂ est un radical azoté non cationique et représente un radical amine primaire (-NH₂), secondaire (-NHR) ou tertiaire (-NR₂) où R, identiques ou non, représentent un radical aliphatique, linéaire ou ramifié, saturé ou non, en C₁-C₂₂ , de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ; ou les radicaux R peuvent former deux à deux, ensemble avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes, des exemples de tels cycles sont les cycles azétidine, pyrrolidine, pipéridine, pipérazine, morpholine, ledit hétérocycle pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl (C₁-C₆) carbonyle, un radical thio (-SH), un radical thioalkyle (-R-SH) en C₁-C₆, un radical alkyl (C₁-C₆) thio, un radical amino, un radical amino mono ou di-substitué par un radical alkyl (C₁-C₆) , alkyl (C₁-C₆) carbonyle, amido ou alkyl(C₁-C₆)sulfonyle.

14. Dérivés selon l'une quelconque des revendications 8 à 12, dans lesquels R₂ est un radical dérivé d'aminoguanidine.

15. Dérivés selon l'une quelconque des revendications 8 à 12 dans lesquels R₂ représente un radical onium Z correspondant à la formule (VIII) : 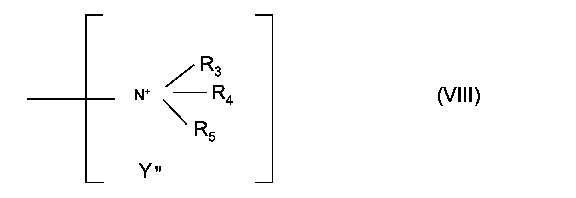
• R₃, R₄ et R₅, pris séparément, identiques ou non, représentent un radical aliphatique, linéaire ou ramifié, saturé ou non, en C₁-C₂₂, de préférence un radical alkyle en C₁-C₂₂ ; un radical alicyclique, saturé ou non, en C₃-C₈ ; un radical monohydroxyalkyle en C₁-C₂₂, de préférence en C₁-C₆ ; un radical polyhydroxyalkyle en C₂-C₂₂, de préférence en C₂-C₆ ; un radical alcoxy (C₁-C₆) alkyle en C₁-C₂₂, de préférence en C₁-C₆ ; un radical aryle ; un radical arylalkyle dont la partie alkyle est en C₁-C₆, comme le radical benzyle par exemple ; un radical amidoalkyle en C₁-C₆ ; un radical trialkyl (C₁-C₆) silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl (C₁-C₆) carbonyle, amido ou alkyl (C₁-C₆) sulfonyle ; ou
• R₃, R₄ et R₅ ensemble, deux à deux, forment, avec l'atome d'azote auxquels ils sont rattachés, un cycle saturé carboné comprenant 3 à 9 chaînons, de préférence 4, 5, 6, 7 ou 8 chaînons, pouvant contenir un ou plusieurs hétéroatomes ; des exemples de ce cycle sont les cycles azétidine, pyrrolidine, pipéridine, pipérazine ou morpholine, ledit hétérocycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆, un radical amido, un radical carboxyle, un radical alkyl (C₁-C₆) carbonyle, un radical thio (-SH), un radical thioalkyle (-R-SH) en C₁-C₆, un radical alkyl(C₁-C₆)thio, un radical amino, un radical amino mono ou di-substitué par un radical alkyl (C₁-C₆) , alkyl (C₁-C₆) carbonyle, amido ou alkyl (C₁-C₆) sulfonyle ;
• Y" est un contre ion.

16. Dérivés selon la revendication 15 dans lesquels dans la formule (VIII) R₃, R₄ et R₅ sont choisis séparément parmi un radical alkyle en C₁-C₆, un radical monohydroxyalkyle en C₁-C₄, un radical polyhydroxyalkyle en C₂-C₄, un radical alcoxy (C₁-C₆) alkyle en C₁-C₄, un radical amidoalkyle en C₁-C₆, un radical trialkyl (C₁-C₆) silanealkyle en C₁-C₆, ou R₃ avec R₄ forment ensemble un cycle azétidine, un cycle pyrrolidine, pipéridine, pipérazine, morpholine, R₅ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle mono ou di-substitué par un radical alkyl(C₁-C₆), alkyl (C₁-C₆) carbonyle, amido ou alkyl (C₁-C₆) sulfonyle ; un radical carbamylalkyle en C₁-C₆ ; un radical trialkyl (C₁-C₆) silanealkyle en C₁-C₆ ; un radical alkyl (C₁-C₆) carboxyalkyle en C₁-C₆ ; un radical alkyl (C₁-C₆) carbonyalkyle en C₁-C₆ ; un radical N-alkyl (C₁-C₆) carbamylalkyle en C₁-C₆ .

17. Dérivés selon la revendication 15 ou 16 dans lesquels dans la formule (VIII) le radical R₂ est un radical trialkylammonium dont les radicaux alkyles peuvent être substitués.

18. Dérivés de paraphénylènediamine selon l'une quelconque des revendications 8 à 12 dans lesquels R₂ représente un radical onium Z correspondant à la formule (IX) : 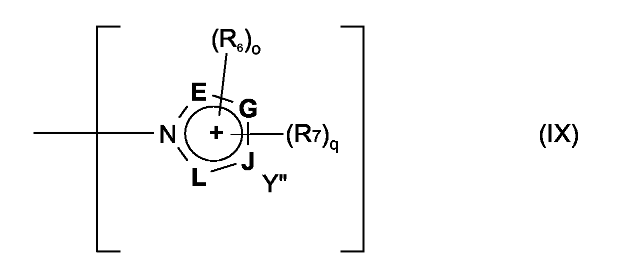 dans laquelle :
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique ;
• q est un nombre entier compris entre 0 et 4 inclus ;
• o est un nombre entier compris entre 0 et 3 inclus ;
• q+o est un nombre entier compris entre 0 et 4 inclus ;
• R₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R₇ sont portés par un atome de carbone ;
• R₆ , identique ou différent, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical benzyle ; étant entendu que les radicaux R₆ sont portés par un azote ;
• Y" est un contre-ion.

19. Dérivés selon la revendication 18 dans lesquels les sommets E, G, J et L forment un cycle pyrrolique, imidazolique, pyrazolique, oxazolique, thiazolique ou triazolique.

20. Dérivés selon la revendication 19 dans lesquels les sommets E, G, J et L forment un cycle imidazolique.

21. Dérivés de paraphénylènediamine selon l'une quelconque des revendications 8 à 12 dans lesquels R₂ représente le radical onium Z correspondant à la formule (X) : 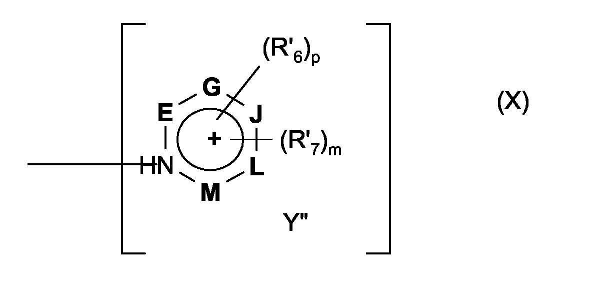 dans laquelle :
• les sommets E, G, J, L et M identiques ou différents, représentent un atome de carbone, d'oxygène, de soufre ou d'azote pour former un cycle choisi parmi les cycles pyridiniques, pyrimidiniques, pyraziniques, triaziniques et pyridaziniques ;
• p est un nombre entier compris entre 0 et 3 inclus ;
• m est un nombre entier compris entre 0 et 5 inclus ;
• p+m est un nombre entier compris entre 0 et 5 inclus ;
• R'₇, identiques ou différents, représentent un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl (C₁-C₆) silanealkyle en C₁-C₆ ; étant entendu que les radicaux R'₇ sont portés par un atome de carbone ;
• R'₆ , identiques ou différents, représente un radical alkyle en C₁-C₆, un radical monohydroxyalkyle en C₁-C₆, un radical polyhydroxyalkyle en C₂-C₆, un radical trialkyl (C₁-C₆) silanealkyle en C₁-C₆, un radical alcoxy (C₁-C₆) alkyle en C₁-C₆, un radical benzyle ; étant entendu que les radicaux R'₆ sont portés par un azote ;
• Y" représente un contre-ion.

22. Dérivés selon la revendication 21 dans lesquels les sommets E, G, J, L et M avec l'azote du cycle forment un cycle pyridinique ou pyrimidinique.

23. Dérivés selon l'une quelconque des revendications 18 à 22 dans lesquels R₆, R₇, R'₇ et R'₆ sont des radicaux alkyles pouvant être substitués.

24. Composition tinctoriale contenant, dans un milieu approprié pour la teinture des fibres kératiniques, au moins un dérivé de paraphénylènediamine de formule (II), dans une configuration (R) ou (S), tel que défini selon l'une quelconque des revendications 8 à 23, à titre de base d'oxydation.

25. Composition selon la revendication 24 comprenant en outre au moins un coupleur choisi parmi les métaphénylènediamines, les méta-aminophénols, les métadiphénols, les coupleurs naphtaléniques, les coupleurs hétérocycliques et leurs sels d'addition.

26. Composition selon l'une quelconque des revendications 24 ou 25 comprenant en outre au moins une base d'oxydation additionnelle autre que les bases d'oxydation de formule (II) choisie parmi les paraphénylènediamines, les bis-phénylalkylènediamines, les para-aminophénols, les ortho-aminophénols, les bases hétérocycliques et leurs sels d'addition.

27. Composition selon l'une quelconque des revendications 24 à 26 dans laquelle la quantité de base d'oxydation de formule (II) est comprise entre 0,001 et 10 %, de préférence entre 0,005 et 6 % en poids environ du poids total de la composition tinctoriale.

28. Composition selon l'une quelconque des revendications 24 à 27 dans laquelle la quantité de chacune des bases d'oxydation additionnelles est comprise entre 0,001 et 10 % en poids environ du poids total de la composition tinctoriale.

29. Composition selon l'une quelconque des revendications 24 à 28 dans laquelle la quantité de chacun des coupleurs est comprise entre 0,001 et 10 %, de préférence entre 0,005 et 6 % en poids environ du poids total de la composition tinctoriale.

30. Procédé de teinture d'oxydation des fibres kératiniques, **caractérisé en ce qu'**on applique sur les fibres une composition tinctoriale telle que définie dans l'une quelconque des revendications 24 à 29 en présence d'un agent oxydant pendant un temps suffisant pour développer la coloration désirée.

31. Procédé selon la revendication 30 dans lequel l'agent oxydant est choisi parmi le peroxyde d'hydrogène, le peroxyde d'urée, les bromates de métaux alcalins, les persels, les peracides et les enzymes oxydases.

32. Dispositif à plusieurs compartiments dans lequel un premier compartiment contient une composition tinctoriale telle que définie dans l'une quelconque des revendications 24 à 29 et un deuxième compartiment contient un agent oxydant.

33. Utilisation de la composition définie aux revendications 24 à 29 pour la teinture de fibres kératiniques.
